# NOUVEAU FASCICULE DE BREVET EUROPEEN

(11) **EP 0 770 377 B2**
(45) Date de publication et mention de la décision concernant l'opposition: **13.04.2005**
(45) Mention de la délivrance du brevet: 28.10.1998
(21) Numéro de dépôt: 96401356.9
(22) Date de dépôt: 20.06.1996
(51) Int. Cl.: A61K 7/42, A61K 7/48

(54) **Composition filtrante à usage cosmétique ou dermatologique contenant un chelateur de fer**
Filtrierende kosmetische oder dermatologische Zusammensetzung enthaltend einen Eisen-Chelat-Bildner
Screening cosmetic or dermatologic composition containing an iron chelator

(30) Priorité: 20.07.1995 FR 9508817
(43) Date de publication de la demande: 02.05.1997
(73) Titulaire: L'OREAL, 75008 Paris (FR)
(72) Inventeur: Simon, Pascal, 94400 Vitry sur Seine (FR); Gagnebien, Didier, 92320 Chatillon (FR)
(74) Mandataire: Casalonga, Axel

(56) Documents cités:
- EP-A- 0 313 305
- EP-A- 0 496 433
- EP-A- 0 496 434
- EP-A- 0 671 160
- WO-A-94/04128
- WO-A-94/04131
- DE-A- 4 342 719
- DE-U- 9 104 777
- DATABASE WPI Week 8927 Derwent Publications Ltd., London, GB; AN 89-197258 XP002001495 & JP-A-01 135 887 (LION CORP.)
- PATENT ABSTRACTS OF JAPAN vol. 11, no. 53 (C-404) [2500] & JP-A-61 215311 (SHISEIDO CO.)

## Description

L'invention se rapporte à une composition notamment cosmétique ou dermatologique, ayant des propriétés filtrantes élevées. Elle permet en particulier de prévenir et/ou de lutter contre le vieillissement de la peau notamment dû aux rayonnements ultraviolets A ainsi que de protéger la peau et/ou les cheveux contre ces rayonnements et contre les radicaux libres. Cette composition se présente sous forme d'une crème blanche onctueuse ou d'un gel pouvant être appliqué sur le visage, le corps et/ou les jambes d'êtres humains ainsi que sur les mains et le cuir chevelu.

Au cours du temps, il apparaît différents signes sur la peau, très caractéristiques du vieillissement, se traduisant en particulier par une modification de la structure et des fonctions cutanées.

Les principaux signes cliniques du vieillissement cutané sont notamment l'apparition de ridules et de rides profondes, en augmentation avec l'âge. On constate en particulier une désorganisation du "grain" de la peau, c'est-à-dire que le micro-relief est moins régulier et présente un caractère anisotrope.

Par ailleurs, le teint de la peau est généralement modifié ; il apparaît plus pâle et plus jaune ; ceci semble être dû essentiellement à une désorganisation de la microcirculàtion (moins d'hémoglobine au niveau du derme papillaire). De plus, de nombreuses taches colorées et/ou plus foncées apparaissent à la surface de la peau, et plus spécialement sur les mains, conférant à la peau une hétérogénéité. En général, ces taches sont dues à une production importante de mélanine dans l'épiderme et/ou le derme de la peau. Dans certains cas, ces taches peuvent devenir cancéreuses. Aussi, on cherche de plus en plus à diminuer, voire éliminer, ces taches. Par ailleurs, il peut exister sur certaines zones de la peau des irritations diffuses et parfois des télangiectasies.

Un autre signe clinique du vieillissement est l'aspect sec et rêche de la peau qui est dû essentiellement à une des-. quamation plus importante ; ces squames, en diffractant les rayons lumineux, participent aussi à l'aspect un peu gris du teint.

On constate enfin une perte de fermeté et de tonicité de la peau qui, comme pour les rides et les ridules, s'explique du moins en partie par une atrophie dermique et épidermique ainsi qu'un applatissement de la formation dermoépidermique ; la peau est moins épaisse et plus flasque, et l'épaisseur de l'épiderme diminue.

On constate donc que les signes cliniques du vieillissement cutané résultent essentiellement d'un dysfonctionnement des principaux mécanismes biologiques intervenant au niveau de la peau.

Ce vieillissement peut être de nature physiologique mais également photo-induit, c'est-à-dire dû à une exposition répétée de la peau à la lumière solaire, et notamment aux rayonnements ultraviolets A. L'action de cette lumière sur les constituants de la peau et sur le sébum sécrété par la peau entraîne en particulier la formation de radicaux libres oxygénés. Or, ces radicaux provoquent des dégats importants, notamment dans les membranes cellulaires (perméabilité des membranes), les noyaux des cellules (mutation par action sur l'ARN ou ADN), et les tissus (nécroses, dégénérescence) ; il est donc nécessaire de protéger la peau contre ces radicaux libres.

Aussi, la composition selon l'invention est une composition apte à prévenir et/ou lutter contre l'apparition du vieillissement et les signes de vieillissement, comme les rides, les ridules, à prévenir et/ou lutter contre les taches de pigmentation de la peau, quelle que soit leur origine, ainsi qu'à protéger la peau notamment par suppression de la formation de radicaux libres oxygénés.

Pour lutter efficacement contre le vieillissement prématuré de la peau, un des moyens connus est d'utiliser un filtre UVA absorbant entre 320 et 400 nm. Ce dernier permet de diminuer l'excès de radicaux libres photo-induits. Toutefois avec la plupart des compositions contenant des filtres UVA, la protection n'est pas totale. Ainsi, lors d'expositions répétées, la quantité résiduelle de radicaux libres persistant malgré la protection par le filtre UVA, peut provoquer à la longue des phénomènes de vieillissement photo-actinique.

Une solution peut consister à augmenter les quantités de filtres UVA, mais il est peu conseillé d'utiliser des taux trop importants de filtres dans les produits cosmétiques de soin quotidien. En effet, avec la plupart des filtres on atteint souvent un indice de protection maximum qu'il est très difficile d'améliorer en augmentant le taux des filtres. En outre, il faut tenir compte du fait que les produits de soin quotidien sont plus fréquement utilisés que d'autres produits saisonniers contenant des filtres comme les produits solaires par exemple, ce qui peut entraîner des inconforts cosmétiques et des problèmes de toxicité par voie topique.

Pour lutter plus efficacement contre le vieillissement photo-actinique, il est donc intéressant de trouver un autre moyen que celui consistant à utiliser des quantités importantes de filtre UVA. En particulier, il est possible d'associer aux filtres UVA des molécules capables de bloquer les réactions en chaîne des radicaux libres avant les étapes ultimes de dégradation des constituants biologiques de la peau. Ces composés sont des agents anti-oxydants et/ou antiradicaux libres.

On sait que les radicaux libres photo-induits proviennent en grande partie de l'oxygène moléculaire. Etant donné qu'il est abondant dans l'organisme et qu'il a une forte capacité à accepter les électrons, les radicaux libres et les espèces activées de l'oxygène qui en dérivent sont les plus nombreux à participer à des réactions radicalaires. On peut dénombrer :
L'oxygène singulet : non radicalaire, très oxydant, très toxique et très rare car d'une durée de vie très courte. Il est le produit de l'excitation de l'oxygène moléculaire par les photons de la lumière.
Le radical anion superoxyde : il est le produit de l'addition d'un électron sur l'oxygène moléculaire. Il peut donner lieu à la production de radicaux libres très réactifs : les radicaux hydroxyles.
Le peroxyde d'hydrogène : non radicalaire mais qui peut donner lieu à la production de radicaux hydroxyles.
Le radical hydroxyle : il est très oxydant donc très réactif et le plus toxique pour les cellules.
On citera en outre les radicaux lipo-peroxydes qui sont les produits d'oxydation des lipides membranaires.
On citera également le fer extracellulaire qui, en réagissant avec le peroxyde d'hydrogène et le radical anion superoxyde accumulés en dehors de la cellule, va favoriser la production du radical hydroxyle.

Le document EP-A-671160 se rapporte à l'utilisation d'acides sulfoniques comme agents pour lutter contre le vieillissement intrinsèque de la peau. Certains de ces acides sont des filtres et se trouvent associés à l'EDTA disodique en une quantité de 0,05 % du poids total de la composition.

Pour lutter efficacement contre les radicaux libres oxygénés et donc contre le photovieillissement, la demanderesse a recherché une association d'un filtre UVA avec une ou plusieurs molécules anti-oxydantes et/ou antiradicalaires, présentant un effet de synergie et notamment un indice de protection supérieur à celui du filtre seul alors que ces molécules anti-oxydantes et/ou antiradicalaires ne présentent aucune activité de filtration du rayonnement UVA.

De façon surprenante, la demanderesse a trouvé qu'en associant un ou plusieurs chélateurs de fer avec un ou plusieurs filtres UVA, on augmentait le pouvoir filtrant de ces derniers et en particulier leur indice de protection (IP).

Le pouvoir filtrant dans le domaine des UVA est représenté par l'indice de protection, noté IP-La détermination de cet indice de protection UVA est basée sur la méthode d'évaluation de la pigmentation immédiate et persistante induite par les UVA (Persistant Pigment Darkening : PPD), décrite par Chardon et col. (Method for the UVA protection assessment of sunscreens based on residual immediate pigment darkening. 20th Annual Meeting of the American Society for Photobiology, Marco island, Florida (USA), June 20-24, 1992).

Ainsi, l'invention a pour objet une nouvelle composition à application topique, destinée notamment à prévenir et/ou lutter contre le vieillissement de la peau et/ou les taches cutanées et/ou protéger la peau et/ou les cheveux contre les rayons ultraviolets et/ou contre les radicaux libres, présentant une efficacité supérieure à celle de l'art antérieur.

Selon une caractéristique principale de invention, la composition contient, dans un milieu acceptable pour une application topique, au moins un filtre absorbant au moins le rayonnement ultraviolet A, comportant au moins une fonction sulfonique évenutellement neutralisée, et au moins un chélateur de fer ne présentant pas de propriété filtrante de ce rayonnement, choisi parmi les sels de l'acide éthylène diamine tétraméthylène phosphonique, le filtre et le chélateur étant en une.quantité telle qu'ils agissent en synergie pour conférer à la composition un indice de protection dans ce rayonnement ultraviolet A, supérieur à celui du filtre seul.
Le ou les chélateurs de fer ont de façon avantageuse une constante d'association avec les ions ferreux ou ferriques supérieure à 10², de préférence supérieure à 10⁶. Comme chélateur de fer, on peut citer notamment les sels pentasodique de l'acide éthyléne diamine tétraméthylène phosphonique. Ils sont utilisés à raison de 0,001 % à 5 % et de préférence de 0,1 % à 2 % du poids total de la composition.

De préférence, la composition de l'invention contient un ou plusieurs autres agents anti-oxydants et/ou antiradicalaires, choisis parmi les agents antilipoperoxyde, les composés régénérateurs de vitamine E oxydé, les agents antiradical hydroxyle, les agents anti-oxygène singulet et les agents antiradical anion superoxyde et leurs associations. Ces composés sont notamment présents en des quantités allant de 0,0001 % à 5 %.

La composition de l'invention contient un ou plusieurs filtres UVA en une quantité efficace pour filtrer ce rayonnement UVA. En pratique, ces filtres représentent de 0,01% à 10% de préférence de 0,1% à 5% du poids total de la composition. Ces filtres sont des molécules à une ou plusieurs fonctions sulfoniques éventuellement neutralisées ou des molécules à une ou plusieurs fonctions sulfonates.

Les bases utilisées pour neutraliser une ou plusieurs fonctions sulfoniques des filtres UVA sont de préférence des bases organiques, généralement utilisées dans le domaine cosmétique, comme la triéthanolamine et l'éthylène diamine.

Les filtres UVA utilisables dans l'invention peuvent être lipophiles ou mieux hydrophiles. Comme filtres utilisables dans l'invention, on peut citer les dérivés sulfonés ou sulfonatés de la benzophénone comme l'acide hydroxy-2-méthoxy-4-benzophénone-5-sulfonique, commercialise notamment par BASF sous le nom d'Uvinul MS-40, ainsi que les dérivés sulfoniques ou sulfonatés du benzylidène camphre.

En particulier, les dérivés de benzylidène camphre utilisables dans l'invention présentent la formule générale (a) suivante : 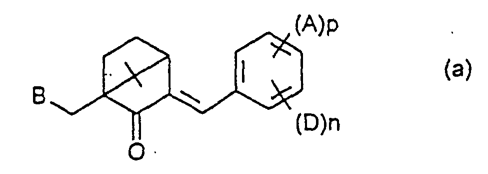 dans laquelle :
B représente -H ou -SO₃H,
0 ≤ p ≤ 1 avec B = -SO₃H quand p = 0,
0≤n≤4.
D représente un ou plusieurs radicaux alkyle ou alcoxy, identiques ou différents quand n ≥ 2, linéaires ou ramifiés contenant de 1 à 18 atomes de carbone environ, un radical halogéno, un radical hydroxyle
A, de préférence en méta ou en para, représente
   soit un radical SO₃H ;
   soit un groupement : 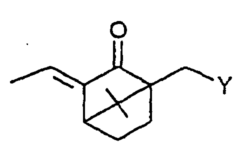
dans lequel Y représente -H ou -SO₃H;
soit un groupement : 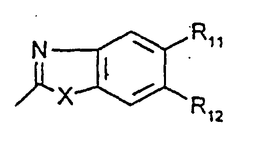 dans lequel :
R₁₁ désigne un atome d'hydrogène, un radical alkyle ou alcoxy, linéaire ou ramifié contenant de 1 à 6 atomes de carbone environ ou le radical -SO₃H, R₁₁ étant -SO₃H lorsque B = -H,
R₁₂ désigne un atome d'hydrogène, un radical alkyle ou alcoxy, linéaire ou ramifié, contenant de 1 à 6 atomes de carbone environ,
X est un atome d'oxygène, de soufre ou un groupement -NR-, R étant un atome d'hydrogène ou un radical alkyle, linéaire ou ramifié, contenant de 1 à 6 atomes de carbone environ,
et dans laquelle au moins une fonction -SO₃H est éventuellement neutralisée.

On peut citer comme exemples particuliers de composés de formule (a) les dérivés de formules (I), (II), (III) suivantes :
*Formule (I) :* 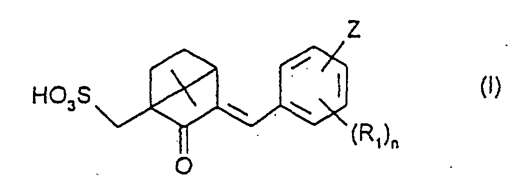 dans laquelle :
- Z, de préférence en position para ou méta, désigne un groupement 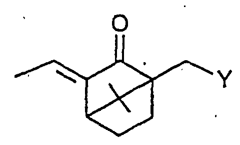 dans lequel Y représente -H ou -SO₃H, éventuellement neutralisé,
- n est égal à 0 ou est un nombre allant de 1 à 4 (0≤n≤4),
- R₁, représente un ou plusieurs radicaux alkyle ou alkoxy, identiques ou différents, linéaires ou ramifiés, contenant de 1 à 4 atomes de carbone environ.

Un composé de formule (I) particulièrement préféré est celui correspondant à n = 0, Z en position para et Y = -SO₃H : l'acide benzène 1,4 [di(3-méthylidènecampho 10-sulfonique)], appelé aussi (selon la nomenclature CTFA - 5ème édition) l'acide téréphtalylidène - di-camphosulfonique.
*Formule (II) :* 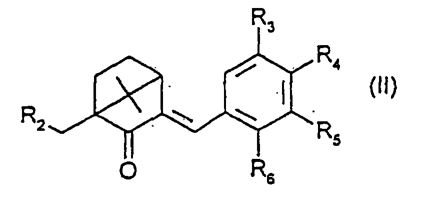 dans laquelle :
- R₂ désigne un atome d'hydrogène ou un radical -SO₃H,
- R₃, R_{4,} R₅ et R₆, identiques ou différents, représentent un groupement hydroxyle, un radical alkyle ayant de 1 à 4 atomes de carbone environ, linéaire ou ramifié, un radical alcényle ayant de 2 à 4 atomes de carbone environ, linéaire ou ramifié, un radical alcoxy ayant de 1 à 4 atomes de carbone, linéaire ou ramifié, un radical alcényloxy ayant de 2 à 4 atomes de carbone, linéaire ou ramifié, un radical halogéno ; de plus un radical R₃ à R₆ seulement peut être un radical -SO₃H, au moins un des radicaux R₃ à R₆ désignant le radical -SO₃H quand R₂ est un atome d'hydrogène. Une ou plusieurs fonctions -SO₃H peuvent aussi être neutralisées.

On peut citer comme exemples particuliers les composés suivants de formule (II) dans laquelle : bull;">
- R₄ désigne le radical -SO₃H en position para du benzylidènecamphre et R₂, R₃, R₅ et R₆ désignent chacun un atome d'hydrogène, c'est-à-dire le 4'-sulfo 3-benzylidènecamphre.
- R₃, R₄, R₅ et R₆ désignent chacun un atome d'hydrogène et R₂ désigne un radical -SO₃H, c'est-à-dire l'acide 3-benzylidène campho-10 sulfonique.
- R₄ désigne un radical méthyle en position para du benzylidènecamphre, R₅ un radical -SO₃H et R₂, R₃ et R₆ représentent un atome d'hydrogène, c'est-à-dire le 4'-méthyl 3'-sulfo 3-benzylidènecamphre.
- R₄ désigne un atome de chlore en position para du benzylidènecamphre, R₅ un radical -SO₃H et R₂, R₃ et R₆ représentent un atome d'hydrogène, c'est-à-dire le 4'-chloro 3'-sulfo 3-benzylidènecamphre.
- R₄ désigne un radical méthyle en position para du benzylidènecamphre, R₃, R₅ et R₆ désignent un atome d'hydrogène et R₂ désigne un radical -SO₃H, c'est-à-dire l'acide 4'-méthyl 3-benzylidène campho 10-sulfonique.
- R₂ représente un radical -SO₃H, R₃ est un radical méthyle, R₄ un atome d'hydrogène, R₅ un radical tertiobutyle, R₆ un radical hydroxyle, c'est-à-dire l'acide 3'-t-butyl 2'-hydroxy 5'-méthyl-3-benzylidène campho-10-sulfonique.
- R₂ représente un radical -SO₃H, R₃ est un radical méthoxy, R₄ un atome d'hydrogène, R₅ un radical tertiobutyle, R₆ un radical hydroxyle, c'est-à-dire l'acide 3'-t-butyl 2'-hydroxy 5'-méthoxy-3-benzylidène campho-10-sulfonique.
- R₂ représente un radical -SO₃H, R₃ et R₅ désignent chacun un radical tertiobutyle, R₄ un radical hydroxyle, R₆ un atome d'hydrogène, c'est-à-dire l'acide 3',5'-diterbutyl 4'-hydroxy-3-benzylidène campho-10-sulfonique.
- R₄ représente un radical méthoxy en para, R₅ représente -SO₃H, les radicaux R₂, R₃ et R₆ représentent H, c'est-à-dire le 4'-méthoxy 3'-suffo-3-benzylidène camphre.
- R₂ désigne un radical -SO₃H, R₃ et R₆ représentent H, R₄ et R₅ formant un radical méthylènedioxy, c'est-à-dire l'acide 3',4'-méthylènedioxy-3-benzylidène campho-10-sulfonique.
- R₂ représente un radical -SO₃H, R₄ un radical méthoxy et les radicaux R₃, R₅ et R₆ représentent H, c'est-à-dire l'acide 4'-méthoxy-3-benzylidène campho-10-sulfonique.
- R₂ représente un radical -SO₃H, R₄ et R₅ sont tous deux un radical méthoxy et les radicaux R₃ et R₆ représentent H, c'est-à-dire l'acide 3',4'-diméthoxy-3-benzylidène campho-10-sulfonique.
- R₂ représente un radical -SO₃H, R₄ est un radical n-butoxy et les radicaux R₃, R₅ et R₆ représentent un atome d'hydrogène, c'est-à-dire l'acide 4'-n.butoxy-3-benzylidène campho-10-sulfonique.
- R₂ représente un radical -SO₃H, R₄ est un radical n-butoxy, R₅ est un radical méthoxy et R₃ et R₆ désignent tous deux un atome d'hydrogène, c'est-à-dire l'acide 3'-méthoxy 4'-n.butoxy-3-benzylidéne campho-10-sulfonique.
*Formule (III) :* 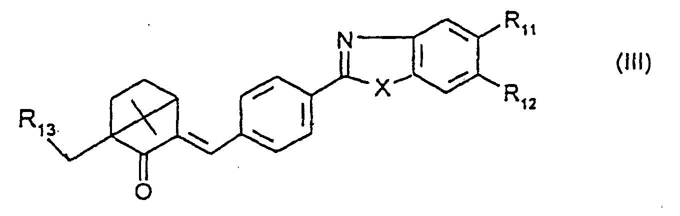 dans laquelle :
- R₁₁ désigne un atome d'hydrogène, un radical alkyle ou alcoxy, linéaire ou ramifié, contenant de 1 à 6 atomes de carbone environ ou un radical -SO₃H,
- R₁₂ désigne un atome d'hydrogène, un radical alkyle ou alcoxy, linéaire ou ramifié, contenant de 1 à 6 atomes de carbone environ,
- R₁₃ désigne un atome d'hydrogène ou un radical -SO₃H,
- l'un au moins des radicaux R₁₁ et R₁₃ désignant un radical -SO₃H,
- X est un atome d'oxygène ou de soufre ou un groupement -NR-, R étant un atome d'hydrogène ou un radical alkyle, linéaire ou ramifié, contenant de 1 à 6 atomes de carbone environ.

On peut citer comme exemple particulier de composé de formule (III) : le composé dans lequel X désigne un radical -NH-, R₁₁ désigne un radical -SO₃H, R₁₂ et R₁₃ désignent tous deux un atome d'hydrogène, c'est-à-dire l'acide 2-[4-(camphométhylidène) phényl] benzimidazole-5-sulfonique.

Les composés de structures (I), (II), (III) sont décrits dans le brevet US 4 585 597 et les brevets FR 2 236 515, 2 282 426, 2 645 148, 2 430 938, 2 592 380.

On peut citer comme autres exemples de dérivés du benzylidène camphre utilisables dans l'invention les composés de formule générale (b) suivante : 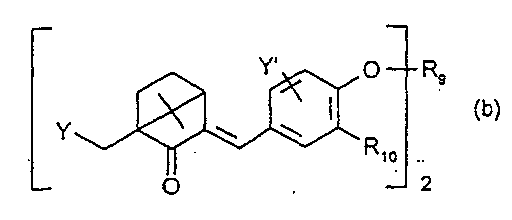 dans laquelle :
- R₉ désigne un radical divalent : -(CH₂)ₘ- ou -CH₂ -CHOH-CH₂-, m étant un nombre entier allant de 1 à 10 (1 ≤ m ≤ 10),
- R₁₀ désigne un atome d'hydrogène, un radical alcoxy contenant de 1 à 4 atomes de carbone environ ou un radical divalent - O - relié au radical R₉ lorsque celui-ci est divalent lui aussi,
- Y et Y' désignent un atome d'hydrogène ou un radical -SO₃H, au moins un de ces radicaux Y ou Y' étant différent de l'hydrogène. Là encore la fonction -SO₃H peut être neutralisée.

On peut citer comme exemple particulier le composé suivant de formule (b) dans laquelle Y représente -SO₃H, Y' est -H, R₁₀ est H et R₉ est -CH₂-CH₂-, c'est à dire l'acide éthylène bis [(4'-oxy benzylidène) 3-campho-10 sulfonique].

On peut utiliser un ou plusieurs agents antilipo-peroxyde en une quantité allant de préférence de 0,05% à 5% et mieux de 0,2% à 3% du poids total de la coomposition. Ces agents sont par exemple la Vitamine E (ou alpha D,L tocophérol) et ses dérivés, ou tout autre composé efficace in vitro au test dit de Béta-carotène ou au test du rancimat, comme le gamma orizanol, des extraits naturels comme les oligomères procyannidoliques (OPC) d'aubépine, de pin ou de raisin, les extraits de noix d'alep, de romarin.

De façon avantageuse, la composition de l'invention contient au moins un filtre UVA tel que l'acide benzène 1,4 [di(3-méthylidène-campho 10-sulfonique)], un chélateur de fer tel que le Dequest 2046 et éventuellement un agent antilipo-peroxyde comme la vitamine E.

La composition de l'invention peut présenter toutes les formes galéniques normalement utilisées pour une application topique telles que les solutions, les gels aqueux ou hydroalcooliques, les émulsions huile-dans-eau ou eau-dans-huile, et plus particulièrement les gouttelettes d'huile dispersées par des sphérules dans une phase aqueuse. Ces sphérules peuvent être des nanoparticules polymériques telles que les nanosphères et les nanocapsules ou mieux des vésicules lipidiques. La composition de l'invention peut se présenter sous forme d'une crème, d'une pommade, d'une lotion ou d'un sérum.

Les huiles utilisables dans l'invention sont celtes généralement utilisées dans les domaines concernés. Elles peuvent être végétales, minérales ou synthétiques, éventuellement siliconées et/ou fluorées.

L'invention peut également contenir des adjuvants hydrophiles ou lipophiles comme des gélifiants, des conservateurs, des opacifiants, des émulsionnants, des coémulsionnants, des neutralisants, des parfums et leurs solubilisants ou peptisants, des matières colorantes comme les colorants et les pigments, des charges, ainsi que des actifs lipophiles ou hydropliles autres que les chélateurs de fer et les filtres chimiques absorbant dans l'ultraviolet A. En particulier, il est possible d'adjoindre à la composition des filtres UVB en vue d'éviter les coups de soleil lors de l'exposition des utilisateurs de cette composition aux rayons solaires.

Les quantités d'huile et d'eau sont généralement celles utilisées dans les domaines considérés et sont fonction de la forme galénique de la composition. Pour une émulsion huile-dans-eau ou une dispersion d'huile dans de l'eau par des sphérules lipidiques, l'huile peut représenter de 0,5% à 60% de préférence de 2 % à 40 % du poids total de la composition.

De même les adjuvants sont utilisés en quantité habituelle et peuvent représenter, au total, de 0,1 % à 20 % en poids. Leur quantité est fonction de leur nature.

La composition de l'invention peut être appliquée par voie topique sur toutes les parties du corps et du visage, y compris sur le cuir chevelu, les jambes et les mains.

L'invention a aussi pour objet une utilisation de la composition définie ci-dessus pour le traitement cosmétique des rides et/ou les ridules de la peau, induites par un rayonnement UVA, ainsi qu'une utilisation de cette composition pour protéger, hydrater et/ou raffermir la peau.

L'invention a aussi pour objet l'utilisation de la composition ci-dessus pour lutter contre le vieillissement photo-induit.

L'invention a encore pour objet une utilisation de la composition définie ci-dessus pour dépigmenter la peau et/ou traiter cosmétiquement les taches cutanées dues au vieillissement, présentes sur le visage et/ou le corps, y compris les mains et le cuir chevelu ainsi que pour la préparation d'une crème destinée au traitement des taches de la peau d'origine pathologique.

L'invention se rapporte aussi à l'utilisation de la composition ci-dessus pour protéger la peau contre les radicaux libres.

L'invention a encore pour objet un procédé de traitement cosmétique et/ou dermatologique de la peau, consistant à appliquer la composition définie ci-dessus sur la peau.

D'autres caractéristiques et avantages de l'invention ressortiront mieux de la description qui suit, donnée à titre illustratif et non limitatif. Dans les exemples ci-après les compositions sont données en % pondéral.

Les tests suivants ont mis en évidence les avantages de la présente invention. Le but des tests a été de montrer l'aptitude des compositions de l'invention à la protection contre les radicaux libres.

A titre d'exemple, on a réalisé la composition (A) suivante :
une émulsion huile-dans-eau contenant un sel penta sodique de l'acide éthylène di-amine tétraméthylène phosphonique (Dequest 2046 de chez Monsanto) et de l'acide benzène 1,4 [di(3-méthylidène-campho 10-sulfonique)]. On a aussi réalisé la composition (B) qui est la même émulsion que la composition (A) mais qui ne contient que le filtre UVA.

De façon surprenante, l'indice de protection UVA (IP) de la composition (A) est supérieur à celui du filtre seul, ce qui montre que la composition de l'invention filtre le rayonnement UVA de façon plus efficace que le filtre seul.

Les valeurs des indices de protection UVA des compositions (A) et (B) ont été mesurées in vivo sur 5 sujets (méthode PPD).

Les résultats sont :
Composition (A) > Composition (B) pour 80 % des sujets (15 % d'augmentation).

La protection dans l'UVA de la composition (A) est donc supérieure, de façon notable, à celle de la composition (B) contenant le filtre seul.

### EXEMPLE 1 : Crème eau-dans-huile pour soin quotidien des rides

### Composition

### A₁

| | |
|---|---|
| - Acide stéarique | 0,4 % |
| - Stéarate de polyéthylène glycol (40.OE) (émulsionnant) | 3,5 % |
| - Mono,di,tri-palmitostéarate de glycéryle (émulsionnant) | 3,0 % |
| - Palmitate d'isopropyle (huile) | 7,0 % |
| - Iso-paraffine (6-8 moles d'isobutylène) hydrogénée (huile) | 6,5 % |

### A₂

| | |
|---|---|
| - Cyclopentadiméthylsiloxane (huile) | 5,0 % |

### B

| | |
|---|---|
| - Eau déminéralisée stérilisée | qsp 100 % |
| - Glycérol (hydratant) | 3,0 % |
| - Chélateur de fer (Dequest 2046) | 2,0 % |
| - Alcool éthylique absolu dénaturé | 10,0 % |
| - Para-hydroxybenzoate de méthyle (conservateur) | 0,2 % |

### C

| | |
|---|---|
| - Acide téréphtalylidène di-camphosulfonique dans l'eau à 33 % | 3,3 % |
| - Triéthanolamine (neutralisant) | 0,7 % |

### Préparation de la phase A₁ + A₂

Les constituants de A₁ sont solubilisés à 80 °C. Lorsque le mélange est limpide, la température est abaissée à 65 °C et on ajoute A₂. Le mélange doit être limpide et homogène. On maintient la température de 65 °C.

### Fabrication

Dans un bécher de fabrication, les constituants de B sont solubilisés à 85 °C-90 °C. Après vérification de la limpidité, la température est ramenée à 65 °C. On réalise l'émulsion, sous agitation, en versant (A₁+A₂) dans B. On poursuit le refroidissement sous agitation. A 40 °C on ajoute la phase C, toujours sous agitation, et on laisse refroidir à 20 °C sous agitation.

On obtient une crème blanche de soin pour protéger quotidiennement la peau des méfaits du rayonnement UV.

### EXEMPLE 2 : Gel quotidien et protecteur contre les rayons solaires

### Composition

### A

| | |
|---|---|
| - Eau déminéralisée stérilisée | qsp 100 % |
| - Glycérine | 3,0 % |
| - Para-hydroxybenzoate de méthyle | 0,2 % |
| - Dequest 2046 | 1,0 % |
| - Gomme de xanthane (épaississant) | 0,2 % |

### B

| | |
|---|---|
| - Parsol MCX (filtre UVB) | 4,0 % |
| - Alkyl benzoate (Finsov TN, société Witco) | 4,0 % |
| - Carbomer (Pemulen TR 2, société Goodrich) | 0,5 % |
| - Triéthanolamine | 0,5 % |

### C

| | |
|---|---|
| - Acide téréphtalylidène di-camphosulfonique dans l'eau à 33 % | 2,3 % |
| - Triéthanolamine (neutralisant) | 0,7 % |

### Fabrication

On prépare la phase A en saupoudrant, sous agitation le gélifiant dans l'ea contenant les ingrédients dissouts. On émulsionne en incorporant la phase B la phase A, sous agitation. On lisse et on laisse refroidir sous agitation à pale lentes. A 35 °C, on ajoute la phase C et on laisse refroidir à 25 °C sous agitation

### EXEMPLE 3 : Crème eau-dans-huile

### Composition

### A

| | |
|---|---|
| - Protegin X (émulsionnant) | 15,00 % |
| - Huile de vaseline | 4,00 % |
| - Huile de tournesol | 6,00 % |

### B

| | |
|---|---|
| - Eau | qsp 100 % |
| - Glycérine | 5,00 % |
| - Chélateur de fer (Dequest 2046) | 2,00 % |
| - Chlorure de sodium | 1,3 % |

### C

| | |
|---|---|
| - Acide téréphtalylidène di-camphosulfonique dans l'eau à 33 % | 6,1 % |
| - Triéthanolamine (neutralisant) | 1,2 % |

### EXEMPLE 4 : Crème huile-dans-eau

### Composition

### A

| | |
|---|---|
| - Stéarate de méthyl-glucose (Glucate SS, Amerchol) | 3,00 % |
| - Acide stéarique | 0,7 % |
| - Huile de vaseline | 5,00 % |
| - Isostéarate d'isostéaryle | 5,00 % |
| - Cyclométhicone | 5,00 % |

### B

| | |
|---|---|
| - Stéarate de sucrose | 1,3 % |
| - Glycérine | 2,00 % |
| - Hexylène glycol | 4,00 % |
| - Eau | qsp 100 % |
| - Dequest 2046 (Chélateur de fer) | 2,00 % |

### C

| | |
|---|---|
| - Acide téréphtalylidène di-camphosulfonique dans l'eau à 33 % | 2,5 % |
| - Triéthanolamine (neutralisant) | 0.5 % |

## Revendications

1. Composition contenant, dans un milieu acceptable pour une application topique, au moins un filtre absorbant au moins le rayonnement ultraviolet A, comportant au moins une fonction sulfonique éventuellement neutralisée, et au moins un chélateur de fer ne présentant pas de propriété filtrante de ce rayonnement choisi parmi les sels de l'acide éthylène diamine tétraméthylène phosphonique, le filtre et le chélateur étant en une quantité telle qu'ils agissent en synergie pour conférer à la composition un indice de protection (IP) dans ce rayonnement ultraviolet A, supérieur à celui du filtre seul.

2. Composition selon l'une des revendications précédentes, **caractérisée en ce que** le chélateur de fer est un sel penta sodique de l'acide éthyléne di-amine tétraméthylène phosphonique.

3. Composition selon l'une des revendications précédentes, **caractérisée en ce que** le chélateur de fer représente de 0,001 % à 5 % et mieux de 0,1 % à 2 % du poids total de la composition.

4. Composition selon l'une des revendications précédentes, **caractérisée en ce que** le filtre représente de 0,01% à 10% et de préférence de 0,1% à 5% du poids total de la composition.

5. Composition selon l'une des revendications précédentes, **caractérisée en ce que** le filtre est un dérivé sulfoné ou sulfonaté du benzylidène camphre.

6. Composition selon l'une des revendications précédentes, **caractérisée en ce que** le filtre présente la formule (I) suivante : 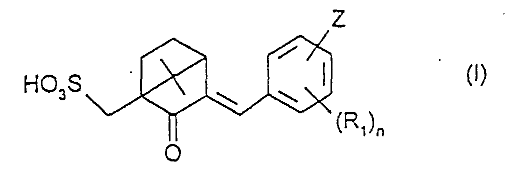 dans laquelle :
- Z désigne un groupement 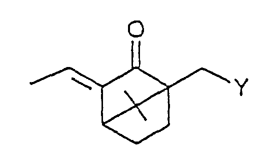 dans lequel Y représente -H ou -SO₃H, éventuellement neutralisé,
- n est égal à 0 ou est un nombre allant de 1 à 4(0 ≤n≤ 4),
- R₁, représente un ou plusieurs radicaux alkyle ou alkoxy, identiques ou différents, linéaires ou ramifiés, contenant de 1 à 4 atomes de carbone.

7. Composition selon l'une des revendications précédentes, **caractérisée en ce que** le filtre est l'acide benzène 1,4 [di(3-méthylidène campho-10-sulfonique)].

8. Composition selon l'une des revendications précédentes, **caractérisée en ce qu'**elle se présente sous forme d'une emulsion, d'un gel ou sous forme d'une dispersion de sphérules.

9. Composition selon l'une des revendications précédentes, **caractérisée en ce qu'**elle contient au moins un agent anti-oxydant et/ou antiradicalaire, choisi parmi les agents anti-lipoperoxyde, les composés régénérateurs de vitamine E oxydé, les agents antiradical hydroxyle, les agents anti-oxygène singulet et les agents antiradical anion superoxyde et leurs associations.

10. Composition selon la revendication 9, **caractérisée en ce que** l'agent antiliperoxyde représente de 0,05 % à 5 % et mieux de 0,2 % à 3 % du poids total de la composition.

11. Composition selon l'une des revendications précédentes, **caractérisée en ce qu'**elle contient, en association, un sel penta sodique de l'acide éthylène diamine tétraméthylène phosphonique et de l'acide benzène 1,4 [di(3-méthylidènecampho 10-sulfonique)].

12. Utilisation de la composition selon l'une des revendications précédentes pour protéger la peau contre les radicaux libres.

13. Utilisation de la composition selon l'une des revendications 1 à 11 pour lutter contre le vieillissement photo-induit.

14. Utilisation de la composition selon l'une des revendications 1 à 11 pour lutter contre les rides et/ou les ridules de la peau, induites par un rayonnement UVA.

15. Procédé de traitement cosmétique de la peau, **caractérisé en ce qu'**il consiste à appliquer sur la peau une composition selon l'une des revendications 1 à 11.

## Patentansprüche

1. Zusammensetzung, die in einem für eine topische Anwendung geeigneten Medium mindestens ein Filter; das mindestens die UV-A-Strahlung absorbiert, das mindestens eine gegebenenfalls neutralisierte Sulfonsäuregruppe enthält, und mindestens einen Chelat-Bildner für Eisen, der keine Filtereigenschaften für diese Strahlung aufweist und unter den Salzen von Ethylendiamintetramethylenphosphonsäure ausgewählt ist, enthält, wobei das Filter und der Chelat-Bildner in einer solchen Menge enthalten sind, daß sie synergistisch wirken, indem sie der Zusammensetzung einen Schutzfaktor (SF) im Bereich dieser UV-A-Strahlung verleihen, der größer ist als der Schutzfaktor des Filters allein.

2. Zusammensetzung nach dem vorhergehenden Anspruch, **dadurch gekennzeichnet, daß** der Chelat-Bildner für Eisen ein Pentanatriumsalz von Ethylendiamintetramethylenphosphonsäure ist.

3. Zusammensetzung nach einem dem vorhergehenden Ansprüche, **dadurch gekennzeichnet, daß** der Chelat-Bildner für Eisen 0,001 bis 5 % und besser 0,1 bis 2 % des Gesamtgewichts der Zusammensetzung ausmacht.

4. Zusammensetzung nach einem der vorhergehenden Ansprüche, **dadurch gekennzeichnet, daß** das Filter 0,01 bis 10 % und vorzugsweise 0,1 bis 5 % des Gesamtgewichts der Zusammensetzung ausmacht.

5. Zusammensetzung nach einem der vorhergehenden Ansprüche, **dadurch gekennzeichnet, daß** das Filter ein Derivat von Benzylidencampher mit Sulfonsäuregruppe(n) oder Sulfonatgruppe(n) ist.

6. Zusammensetzung nach einem der vorhergehenden Ansprüche, **dadurch gekennzeichnet, daß** das Filter die folgende Formel (I) 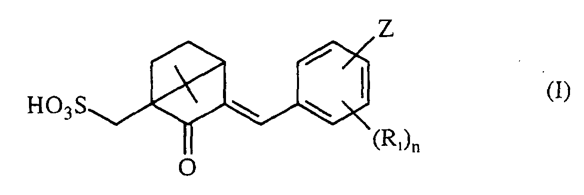 aufweist, in der bedeuten:
- Z eine Gruppe 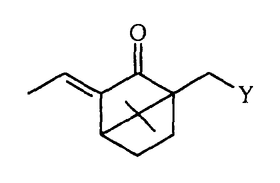 in der Y-H oder eine Gruppe -SO₃H, die gegebenenfalls neutralisiert ist,
- n 0 oder eine Zahl von 1 bis 4 (0 ≤ n ≤ 4),
- R₁ eine oder mehrere geradkettige oder verzweigte, gleiche oder verschiedene Alkyl- oder Alkoxygruppen mit 1 bis 4 Kohlenstoffatomen.

7. Zusammensetzung nach einem der vorhergehenden Ansprüche, **dadurch gekennzeichnet, daß** es sich bei dem Filter um Benzol-1,4-di-(3-methylidencampher-10-sulfonsäure) handelt.

8. Zusammensetzung nach einem der vorhergehenden Ansprüche, **dadurch gekennzeichnet, daß** sie in Form einer Emulsion, eines Gels oder in Form einer Dispersion von Kügelchen vorliegt.

9. Zusammensetzung nach einem der vorhergehenden Ansprüche, **dadurch gekennzeichnet, daß** sie mindestens ein Antioxidationsmittel und/oder ein Mittel gegen Radikale enthält, das unter Mitteln gegen Lipoperoxide, Verbindungen, die oxidiertes Vitamin E regenerieren, Mitteln gegen Hydroxyl-Radikale, Mitteln gegen Singulettsauerstoff und Mitteln gegen Superoxid-Radikalanionen und deren Kombinationen ausgewählt ist.

10. Zusammensetzung nach Anspruch 9, **dadurch gekennzeichnet, daß** das Mittel gegen Lipoperoxide in einem Anteil von 0,05 bis 5 % und besser 0,2 bis 3 % des Gesamtgewichts der Zusammensetzung enthalten ist.

11. Zusammensetzung nach einem der vorhergehenden Ansprüche, **dadurch gekennzeichnet, daß** sie ein Pentanatriumsalz von Ethylendiamintetramethylenphosphonsäure in Kombination mit Benzol-1,4-di-(3-methylidencampher-10-sulfonsäure) enthält.

12. Verwendung der Zusammensetzung nach einem der vorhergehenden Ansprüche für den Schutz der Haut vor freien Radikalen.

13. Verwendung der Zusammensetzung nach einem der Ansprüche 1 bis 11 für die Bekämpfung der lichtbedingten Alterung.

14. Verwendung der Zusammensetzung nach einem der Ansprüche 1 bis 11 für die Bekämpfung der Falten und/oder Fältchen der Haut, die durch UV-A-Strahlung hervorgerufen werden.

15. Verfahren für die kosmetische Behandlung der Haut, **dadurch gekennzeichnet, daß** auf die Haut eine Zusammensetzung nach einem der Ansprüche 1 bis 11 aufgetragen wird.

## Claims

1. Composition containing, in a medium acceptable for topical application, at least one screening agent which absorbs at least ultraviolet A radiation, including at least one optionally neutralized sulpho functional group, and at least one iron chelator which does not exhibit the property of screening this radiation and is chosen from the salts of ethylenediaminetetramethylenephosphonic acid, the screening agent and the chelator being in an amount such that they act in synergy in order to impart to the composition a protection factor (PF) in this ultraviolet A radiation which is greater than that of the screening agent alone.

2. Composition according to the preceding claim, **characterized in that** the iron chelator is a pentasodium salt of ethylenediaminetetramethylenephosphonic acid.

3. Composition according to one of the preceding claims, **characterized in that** the iron chelator represents from 0.001 % to 5 % and, better, from 0.1 % to 2 % of the total weight of the composition.

4. Composition according to one of the preceding claims, **characterized in that** the screening agent represents from 0.01 % to 10 % and preferably from 0.1 to 5 % of the total weight of the composition.

5. Composition according to one of the preceding claims, **characterized in that** the screening agent is a sulpho or sulphonate derivative of benzylidenecamphor.

6. Composition according to one of the preceding claims, **characterized in that** the screening agent has the following formula (I): 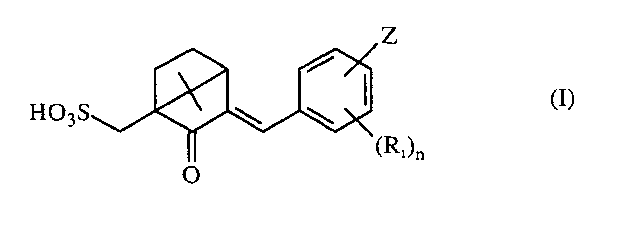 in which:
Z denotes a group 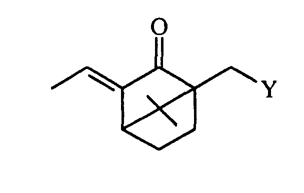
in which Y represents -H or SO₃H, which is optionally neutralized,
- n is equal to 0 or is a number ranging from 1 to 4 (0 ≤ n ≤ 4),
- R₁ represents one or more linear or branched alkyl or alkoxy radicals, which may be identical or different, containing from 1 to 4 carbon atoms.

7. Composition according to one of the preceding claims, **characterized in that** the screening agent is benzene-1,4-[di(3-methylidene-10-camphorsulphonic)] acid.

8. Composition according to one of the preceding claims, **characterized in that** it is provided in the form of an emulsion or of a gel or in the form of a dispersion of spherules.

9. Composition according to one of the preceding claims, **characterized in that** it contains at least one antioxidizing and/or antiradical agent chosen from agents for combating lipoperoxide radicals, compounds for regenerating oxidized viamin E, agents for combating the hydroxyl radical, agents for combating singlet oxygen and agents for combating the superoxide radical anion, and their combinations.

10. Composition according to Claim 9, **characterized in that** the agent for combating lipoperoxide radicals represents from 0.05 % to 5 % and, better, from 0.2 % to 3 % of the total weight of the composition.

11. Composition according to one of the preceding claims, **characterized in that** it contains, in combination, a pentasodium salt of ethylenediaminetetramethylenephosphonic acid and benzene-1,4-[di(3-methylidene-10-camphorsulphonic)] acid.

12. Use of the composition according to one of the preceding claims for protecting the skin against free radicals.

13. Use of the composition according to one of Claims 1 to 11 for combating photoinduced ageing.

14. Use of the composition according to one of Claims 1 to 11 for combating wrinkles and/or fine lines of the skin induced by UVA radiation.

15. Cosmetic treatment process for the skin, **characterized in that** it consists in applying a composition according to one of Claims 1 to 11 to the skin.
